# EUROPEAN PATENT APPLICATION

(11) **EP 4 293 110 A1**
(43) Date of publication of application: **20.12.2023**
(21) Application number: 22305886.8
(22) Date of filing: 17.06.2022
(51) Int. Cl.: C12N 5/078

(54) **PROCESS FOR OBTAINING FUNCTIONAL LYMPHOCYTES CELLS**

(71) Applicant: NANTES UNIVERSITÉ, 44000 Nantes (FR); Institut National de la Santé et de la Recherche Médicale, 75013 Paris (FR)
(72) Inventor: PROVIN, Nathan, 44200 Nantes (FR); GIRAUD, Matthieu, 44300 Nantes (FR)
(74) Representative: LLR

(57) **Abstract**

The invention relates to the use of a composition comprising the following compounds: Activin A, BMP4, CHIR99, EGF, FGF 8, FGF 10, IGF1, LY3, Noggin, retinoic acid and Y27, for implementing a differentiation process, preferably in vitro or ex vivo, of an induced pluripotent stem cell or iPSc, into a functional thymic epithelial progenitor or TEP.

## Description

The invention relates to a process for obtaining functional Lymphocyte cells, and the cells thereof.

The thymus provides the essential microenvironment for T-cell development and maturation. Thymic epithelial cells (TECs), which are composed of thymic cortical epithelial cells (cTECs) and thymic medullary epithelial cells (mTECs), have been well documented to be critical for these tightly regulated processes.

While TEC populations are known to be key regulators of distinct T cell development programs, recent studies have uncovered significant new TEC heterogeneity that must be considered in relation to understanding of microenvironmental control of T cell development. Significantly, therapeutic interventions can also be detrimental to thymus function. Such clinical treatments include ablative preconditioning used in the treatment of cancer, which then impairs T cell mediated immune reconstitution following bone marrow transplantation. Consequently, studying thymic epithelial cells in both health and disease states is important to understand how thymus function is controlled, and how it might be manipulated for therapeutic benefit. Recently, important advances have been made in understanding the biology of thymic epithelium, including the developmental pathways that give rise to distinct cortical and medullary epithelial lineages. Furthermore, there is progress in how newly identified heterogeneity in thymic epithelium may map to functional specialization in thymic microenvironments.

In vitro generation of functional TECs or TECs progenitors (TEPs) from human pluripotent stem cells (hPSCs) could generate cells, tissues or organs which aid in T cell reconstitution in patients with thymic dysfunction due to congenital disorders such as DiGeorge syndrome and acquired dysfunction due to HIV infection, high dose chemotherapy and radiotherapy treatment, graft-vs-host disease and long-term immunosuppressive therapy combined with advanced age, which in itself results in poor thymopoietic function. As the number of TECs in human adult thymi is limited and reliable methods of expanding them from post-natal thymi have been elusive, generating TECs from pluripotent stem cells (PSCs) is an important goal. Creating an in vitro protocol for tightly controlled differentiation of hPSCs to TECs requires precise knowledge and application of developmental temporal and cytokine cues. While the generation of functional TEPs from murine or human PSCs that support murine or human T cell development has been described after transplantation of the TEPs under the kidney capsule in mice, reconstitution of high levels of naïve human T cells in vitro by potent PSCderived TECs has not been demonstrated. Thus, there is a need in the art for a method to generate TEPs and TECs that are able to support efficient T cells development in vitro.

The ability to generate functional TECs from human pluripotent stem cells, would have important applications in modeling human immune responses in mice, and in modeling and treating thymus deficiency syndromes, such as DiGeorge syndrome, Nude syndrome, and immunodeficiency complicating bone marrow transplantation for leukemia. Cells could also be used clinically for cell-therapy and transplanted in patients to achieve T cell reconstitution, or generating immune tolerance to prevent graft rejection after an organ transplantation, or for recovering an impaired thymic functionality due to injuries or aging.

International application WO2020205859 discloses a method to induce in vitro differentiation of human pluripotent stem cells (hPSCs) including embryonic stem cells (ESCs) and induced pluripotent stem cells (iPSCs) into thymic epithelial cell progenitors (TEC progenitors), wherein the thymic epithelial cells (TECs) or thymic epithelial cell progenitors (TEPs) are capable of generating thymic organs and T cells in vivo. This application teaches differentiation culture media, supplemented with Noggin, retinoic acid, FGF8b, sonic hedgehog, BMP, and YM155. In this application the inventors shows that the generation of TEC progenitors and their subsequent maturation in TEC can be carried out after transplantation in mice.

However, *in vitro* functional thymopoieisis, i.e. T cells generation, for use in clinical application and therapies remains cannot be carried out easily in view of the mandatory animal implantation.

Thus there is a need to provide an invitro process for producing functional T cells from iPSC, for subsequente free use in therapy.One aim of the invention is to provide a method for producing thymic epithelial cells and thymic epithelial progenitors, from pluripotent cells, and in particular from iPSC.

Another aim of the invention is to provide a method for treating pathologies involving thymus defects.

The invention relates to a use of a composition comprising the following compounds: Activin A, BMP4, CHIR99, EGF, FGF 8, FGF 10, IGF1, LY3, Noggin, retinoic acid and Y27,
for implementing a differentiation process, preferably in vitro or ex vivo, of an induced pluripotent stem cell or iPSc, into a functional thymic epithelial progenitor or TEP.

The invention is based on the unexpected observation made by the inventors that a specific combination of growth factor allows to differentiate induced pluripotent stem cells into functional thymic epithelial progenitors or TEP. Compared to the art, the TEP obtained according to the invention are fully functional and can be easily and efficiently differentiated into mature TEC, i.e medullary TEC (mTEC) and cortical TEC (cTEC). Thymoid bodies can also be obtained by using the above mentioned composition, further to the differentiation process.

As used herein, the terms "differentiation" and "cell differentiation" refer to a process by which a less specialized cell (i.e., stem cell) develops or matures or differentiates to possess a more distinct form and/or function into a more specialized cell or differentiated cell, (i.e., thymic epithelial cell).

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that not all progenies will have precisely identical DNA content, due to deliberate or inadvertent mutations. Mutant progeny that has the same function or biological activity as screened for in the originally transformed cell are included. Where distinct designations are intended, it will be clear from the context.

As used herein, the term "induced pluripotent stem cells" commonly abbreviated as iPS cells or iPSCs or IPScs, refers to a type of pluripotent stem cell artificially generated from a non- pluripotent cell, typically an adult somatic cell, or terminally differentiated cell, such as fibroblast, a hematopoietic cell, a myocyte, a neuron, an epidermal cell, or the like.

IPSc are non-naturally occurring cells that are genetically modified in order to enforce expression of some genes that revert differentiation program, such that the specialized acquired function of a differentiated cell disappears whereas pluripotency is acquired.

Pluripotency describes the ability of a cell to develop into the three primary germ cell layers of the early embryo and therefore into all cells of the adult body, but not extra-embryonic tissues such as the placenta. Embryonic stem cells and induced pluripotent stem cells are characterized by their pluripotency. Only totipotent cells are able to provide all the differentiated cells. These totipotent cells are not covered by the present invention.

iPSCs are typically derived by introducing products of specific sets of pluripotencyassociated genes, or "reprogramming factors", into a given cell type. The original set of reprogramming factors (also dubbed Yamanaka factors) are the transcription factors Oct4 (Pou5f1), Sox2, Klf4 and cMyc. While this combination is most conventional in producing iPSCs, each of the factors can be functionally replaced by related transcription factors, miRNAs, small molecules, or even non-related genes such as lineage specifiers.

iPSC differentiation is typically a slow and inefficient process, taking 1-2 weeks for mouse cells and 3-4 weeks for human cells, with efficiencies around 0.01-0.1%. However, considerable advances have been made in improving the efficiency and the time it takes to obtain iPSCs. Upon introduction of reprogramming factors, cells begin to form colonies that resemble pluripotent stem cells, which can be isolated based on their morphology, conditions that select for their growth, or through expression of surface markers or reporter genes. More details are given below.

In the invention, the following compounds are used:
**Activin A** is a member of the TGF-β superfamily, has been recognized as a multifunctional cytokine, expressed in a wide range of tissues and cells with roles in the regulation of inflammation, fibrosis, and wound repair, that promotes atherogenesis by inhibiting foam cell formation and neointimal hyperplasia and HF
**BMP4,** is a member of the bone morphogenetic protein family which is part of the transforming growth factor-beta superfamily. The superfamily includes large families of growth and differentiation factors. BMP4 is highly conserved evolutionarily. BMP4 is found in early embryonic development in the ventral marginal zone and in the eye, heart blood and otic vesicle
**CHIR99,** or CHIR99021, or -[[2-[[4-(2,4-Dichlorophenyl)-5-(5-methyl-1H-imidazol-2-yl)-2-pyrimidinyl]amino]ethyl]amino]-3-pyridinecarbonitrile is an aminopyrimidine derivative of formula 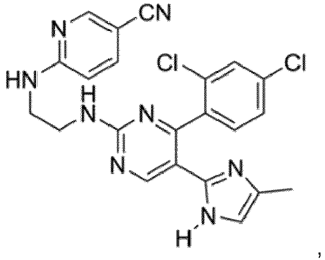 with a CAS number 252917-06-9. CHIR99021 promotes self-renewal potential of embryonic stem cells (ESCs) of mice by inhibiting glycogen synthase kinase-3 (GSK-3) activity and potentiates the upregulation of β-catenin and c-Myc functions. CHIR99021 promotes self-renewal of ESCs by modulating transforming growth factor β (TGF-β), Notch and mitogen-activated protein kinases (MAPK) signaling pathways. CHIR99021 is an agonist of wingless/integrated (wnt) signalling, upregulates cyclinA expression and promotes cell proliferation in non-small-cell lung cancer (NSCLC) cell lines.
**EGF,** Epidermal growth factor, is a protein that stimulates cell growth and differentiation by binding to its receptor, EGFR. In humans, EGF has 53 amino acids (sequence NSDSECPLSHDGYCLHDGVCMYIEALDKYACNCVVGYIGERCQYRDLKWWELR), with a molecular mass of around 6 kDa. It contains three disulfide bridges (Cys6-Cys20, Cys14-Cys31, Cys33-Cys42).

**FGF 8,** Fibroblast Growth Factor 8, is important and necessary for setting up and maintaining the midbrain/hindbrain border (or mesencephalon/met-encephalon border) which plays the vital role of "organizer" in development, like the Spemann "organizer" of the gastrulating embryo. FGF 8 is expressed in the region where Otx2 and Gbx2 cross inhibit each other and is maintained expression by this interaction. Once expressed, the FGF 8 induces other transcription factors to form cross-regulatory loops between cells, thus the border is established. Through development, FGF 8 regulates growth and differentiation of progenitor cells in this region to produce ultimate structure of midbrain and hindbrain. It has been demonstrated that FGF8 is sufficient to induce the repatterning of midbrain and hindbrain structure.

**FGF 10,** Fibroblast Growth Factor 10, is a member of the fibroblast growth factor (FGF) family. FGF family members possess broad mitogenic and cell survival activities, and are involved in a variety of biological processes, including embryonic development, cell growth, morphogenesis, tissue repair, tumor growth and invasion. Fibroblast growth factor 10 is a paracrine signaling molecule seen first in the limb bud and organogenesis development. FGF10 starts the developing of limbs and is involved in the branching of morphogenesis in multiple organs such as the lungs, skin, ear and salivary glands. FGF10 signaling is required for epithelial branching. Therefore, all branching morphogen organs such as the lungs, skin, ear and salivary glands required the constant expression of FGF10. This protein exhibits no activity for fibroblasts.

**IGF-1,** Insulin-like growth factor 1, also called somatomedin C, is a hormone similar in molecular structure to insulin which plays an important role in childhood growth, and has anabolic effects in adults.

**LY3,** i.e. LY364947, also called 4-[3-(2-pyridinyl)-1H-pyrazol-4-yl]-quinoline, is a compound of formula 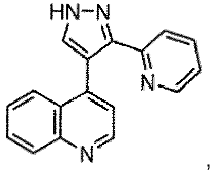 with a CAS number 396129-53-6. This compound is a is a potent ATPcompetitive inhibitor of TGFβR-I. In combination with valproic acid, this compound can replace SOX2 in reprogramming of mouse embryonic fibroblasts transduced with OCT4, KLF4 and c-MYC.

**Noggin,** also known as NOG, is a protein that is involved in the development of many tissues, including nerve tissue, muscles, and bones. The amino acid sequence of human noggin is highly homologous to that of rat, mouse, and Xenopus (an aquatic-frog genus). Noggin is an inhibitor of several bone morphogenetic proteins (BMPs): it inhibits at least BMP2, BMP4, BMP5, BMP6, BMP7, BMP13, and BMP14.

**Retinoic acid,** (also called all-trans-retinoic acid or RA) is a metabolite of vitamin A1 (all-trans-retinol) that mediates the functions of vitamin A1 required for growth and development. All-trans-retinoic acid is required in chordate animals, which includes all higher animals from fish to humans. During early embryonic development, all-trans-retinoic acid generated in a specific region of the embryo helps determine position along the embryonic anterior/posterior axis by serving as an intercellular signaling molecule that guides development of the posterior portion of the embryo. RA acts through Hox genes, which ultimately control anterior/posterior patterning in early developmental stages.

**Y-27632,** hereafter Y27, (*R*)-(+)-*trans*-4-(1-Aminoethyl)-N-(4-Pyridyl)cyclohexanecarboxamide dihydrochloride is a compound of following formula: 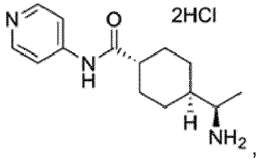 with CAS Number 129830-38-2. Y27 is a cell-permeable, highly potent and selective inhibitor of Rho-associated, coiled-coil containing protein kinase (ROCK). Y27 has been described to enhance survival of human embryonic stem (ES) cells when they are dissociated to single cells by preventing dissociation-induced apoptosis (anoikis), thus increasing their cloning efficiency, to improves embryoid body formation using forcedaggregation protocols, to increase the survival of cryopreserved single human ES cells after thawing and to block apoptosis of mouse ES-derived neural precursors after dissociation and transplantation. Y27 was also involved in direct lineage reprogramming of fibroblasts to mature neurons, in combination with CHIR99021. Finally, Y27 is known to improve survival of human ES cell monolayers at the initiation of differentiation protocols.

Advantageously, the above compounds are used simultaneously, separately, or sequentially.

It is more advantageous that the compounds used to induce differentiation of iPSC into TEP be used sequentially.

Advantageously, the invention relates to the use as defined above, wherein the differentiating process takes place for at least 14 days.

In order to carry out the differentiation process from iPSC to TEP, it is mandatory to use the composition as defined above during at least 14 days.

More advantageously, the compounds contained in the composition as defined above can be used sequentially, at least one time, during the above mentioned at least 14 days.

For instance, the Y27 compound can be used at the beginning of the process of differentiation, then the CHIR99 compound and Actinin A, further retinoic acid and FGF8, further BMP4 and the LY3 compound, then FGF10, IGF1 and EGF.

Advantageously, the invention relates to the use as defined above, wherein the iPSc expresses *OCT4* and *NANOG* genes.

The generation of induced pluripotent cells is crucially dependent on the transcription factors used for the induction. Oct-3/4 and certain products of the Sox gene family (Sox1, Sox2, Sox3, and Sox15) have been identified as crucial transcriptional regulators involved in the induction process whose absence makes induction impossible. Additional genes, however, including certain members of the Klf family (Klf1, Klf2, Klf4, and Klf5), the Myc family (c-myc, L-myc, and N-myc), Nanog, and LIN28, have been identified to increase the induction efficiency.

**Oct-3/4 (Pou5f1):** Oct-3/4 is one of the family of octamer ("Oct") transcription factors, and plays a crucial role in maintaining pluripotency. The absence of Oct-3/4 in Oct-3/4+ cells, such as blastomeres and embryonic stem cells, leads to spontaneous trophoblast differentiation, and presence of Oct-3/4 thus gives rise to the pluripotency and differentiation potential of embryonic stem cells. Various other genes in the "Oct" family, including Oct-3/4's close relatives, Oct1 and Oct6, fail to elicit induction, thus demonstrating the exclusiveness of Oct-3/4 to the induction process. However a team headed by Hans Schöler (who discovered the Oct4 gene back in 1989) showed that Oct4 overexpression during reprogramming causes epigenetic changes deteriorating the quality of iPSCs.

**Sox family:** The Sox family of transcription factors is associated with maintaining pluripotency similar to Oct-3/4, although it is associated with multipotent and unipotent stem cells in contrast with Oct-3/4, which is exclusively expressed in pluripotent stem cells. While Sox2 was the initial gene used for induction, other transcription factors in the Sox family have been found to work as well in the induction process. Sox1 yields iPS cells with a similar efficiency as Sox2, and genes Sox3, Sox15, and Sox18 also generate iPS cells, although with decreased efficiency.

**Klf family:** Klf4 of the Klf family of transcription factors was initially identified. As a factor for the generation of mouse iPS cells and was demonstrated as a factor for generation of human iPS cells. Klf2 and Klf4 were found to be factors capable of generating iPS cells, and related genes Klf1 and Klf5 did as well, although with reduced efficiency.

**Myc family:** The Myc family of transcription factors are proto-oncogenes implicated in cancer. It was demonstrated that c-myc is a factor implicated in the generation of mouse iPS cells and it was a factor implicated in the generation of human iPS cells. However, usage of the "myc" family of genes in induction of iPS cells is troubling for the eventuality of iPS cells as clinical therapies, as 25% of mice transplanted with c-myc-induced iPS cells developed lethal teratomas. N-myc and L-myc have been identified to induce pluripotency instead of c-myc with similar efficiency.

**Nanog:** In embryonic stem cells, Nanog, along with Oct-3/4 and Sox2, is necessary in promoting pluripotency. It has also been reported that it is possible to generate iPS cells with Nanog as one of the factors.

**LIN28:** LIN28 is an mRNA binding protein expressed in embryonic stem cells and embryonic carcinoma cells associated with differentiation and proliferation. It was demonstrated that LIN28 is a factor in iPSC generation in combination with OCT4, SOX2, and NANOG.

**Glis1:** Glis1 is transcription factor that can be used with Oct-3/4, Sox2 and Klf4 to induce pluripotency. It poses numerous advantages when used instead of C-myc.

Advantageously, the invention relates to the use of the following compounds: Activin A, BMP4, CHIR99, EGF, FGF 8, FGF 10, IGF1, LY3, Noggin, retinoic acid Y27, RANKL, IL-7, FLT3, SCF and Glutamax
for implementing a differentiation process, preferably in vitro or ex vivo, of an induced pluripotent stem cell or iPSc, into a functional thymic epithelial cell.

In other words, the invention relates to the use of a composition comprising BMP4, FGF 8, FGF 10, IGF1, EGF, RANKL, IL7, FLT3, SCF and glutamax, for implementing a differentiation process, preferably in vitro or ex vivo, of TEP cells as defined above (i.e. from TEP obtained from the differentiation of an iPSC) into TEC.

The inventors discovered that the TEP obtained as defined above can be efficiently differentiated into thymic organoids, or TEC, by using the above mentioned composition.

**RANKL,** Receptor activator of nuclear factor kappa-B ligand, is known as a type II membrane protein and is a member of the tumor necrosis factor (TNF) superfamily. RANKL has been identified to affect the immune system and control bone regeneration and remodeling. RANKL is an apoptosis regulator gene, a binding partner of osteoprotegerin (OPG), a ligand for the receptor RANK and controls cell proliferation by modifying protein levels of Id4, Id2 and cyclin D1. RANKL is expressed in several tissues and organs including: skeletal muscle, thymus, liver, colon, small intestine, adrenal gland, osteoblast, mammary gland epithelial cells, prostate and pancreas. Variation in concentration levels of RANKL throughout several organs reconfirms the importance of RANKL in tissue growth (particularly bone growth) and immune functions within the body.

**IL7,** Interleukine 7 or IL-7, stimulates the differentiation of multipotent (pluripotent) hematopoietic stem cells into lymphoid progenitor cells. IL7 also stimulates proliferation of all cells in the lymphoid lineage (B cells, T cells and NK cells). IL7 is important for proliferation during certain stages of B-cell maturation, T and NK cell survival, development and homeostasis.

**FTL3,** or FLT3-L, is the ligand of the tyrosine kinase receptor FMS-like tyrosine kinase 3 also called FLT3. FTL3 is a hematopoietic cytokine that regulates the proliferation of early hematopoietic cells. It has been shown to synergize with a variety of cytokines to stimulate the growth and differentiation of early hematopoietic cells. The FLT3 ligand also stimulates the expansion of monocytes and immature dendritic cells, and induces early B cell lineage differentiation and NK cell growth.

**SCF,** Stem cell factor (also known as SCF, KIT-ligand, KL, or steel factor) is a cytokine that binds to the c-KIT receptor (CD117). SCF plays an important role in the hematopoiesis during embryonic development. Sites where hematopoiesis takes place, such as the fetal liver and bone marrow, all express SCF. SCF may serve as guidance cues that direct hematopoietic stem cells (HSCs) to their stem cell niche (the microenvironment in which a stem cell resides), and it plays an important role in HSC maintenance. SCF has been shown to increase the survival of HSCs in vitro and contributes to the self-renewal and maintenance of HSCs in-vivo. HSCs at all stages of development express the same levels of the receptor for SCF (c-KIT). The stromal cells that surround HSCs are a component of the stem cell niche, and they release a number of ligands, including SCF.

**Glutamax** is an L-Glutamine alternative. Glutamax is a dipeptide L-alanyl-L-glutamine in 0.85 % NaCl.

As for the composition used to allow differentiation of IPSC into TEP cells, the compounds contained in the composition allowing differentiation of TEP into TEC can be used simultaneously, separately or sequentially.

Advantageously, RANKL, IL7, FTL3, SCF and Glutamax are used simultaneously during all the differentiation process. These compounds can be associated with BMP4, FGF 8, FGF 10, IGF1 and EGF, at least for the first steps of the differentiation process from TEP to TEC.

The invention relates to a method for differentiating, in vitro or ex vivo, an iPSc into a functional TEP, the method comprising the following steps:
a) Incubating the iPSc in a culture medium supplemented with a Y27 compound for 24h, to obtain a Y27 culture medium,
b) removing the Y27 compound from the Y27 compound, and adding an Activin A growth factor and a CHIR99 compound for 24h, to obtain an ActA/CHIR99 culture medium,
c) removing the CHIR99 growth from the ActA/CHIR99 culture medium, and maintaining the culture with the Activin A for 48h, to obtain an ActA culture medium,
d) Adding to the ActA culture a Y27 compound for 24h, to obtain a Y27/ActA culture medium,
e) Removing the ActA and the Y27 compound from the Y27/Act1 culture medium, and adding to the Y27/Act1 culture medium a FGF8 growth factor and retinoic acid for 24h, to obtain a RA /F8 culture medium,
f) Adding to the RA/F8 culture medium a Noggin growth factor and a LY3 compound for 48h, to obtain a RA/F8/NOG/LY3 culture medium,
g) Removing the Noggin growth factor from the RA/F8/NOG/LY3 culture medium, and adding a CHIR99 compound and a BMP4 growth factor for 48h, to obtain a RA/F8/LY3/CHIR99/BMP culture medium,
h) Removing from the RA/F8/LY3/CHIR99/BMP culture the LY3 compound for 24h to obtain a RA/F8/CHIR99/BMP culture medium,
i) Modifying the concentration of the BMP4 growth factor and the FGF8 growth factor for 24h in the RA/F8/CHIR99/BMP culture medium,
j) Removing from the RA/F8/CHIR99/BMP culture medium the CHIR99 compound and adding for 72h a FGF10 growth factor, an IGF1 growth factor and an EGF growth factor, to obtain a RA/F8/BMP/F10/IGF1/EGF culture medium, and
k) Recovering the functional TEP from the RA/F8/BMP/F10/IGF1/EGF culture medium, after 72h.

The inventors have shown, further to specific studies of a collection of compounds and growth factors involved in thymic differentiation process, that the sequence of 11 steps is required for obtaining TEP cells which are fully functional and can be used further for initiating another differentiation process in order to obtain TEC cells.

From step b) to step j) reference is made to "adding" or "removing" one or more compound or growth factor. This means that the compound or the growth factors are either
- added to the previous culture, or a new culture medium containing the corresponding compounds and/or growth factor is used with the new compound or growth factor, by replacing the previous one,
- removed from the previous culture medium, or a new culture medium containing the corresponding compounds and/or growth factor is used without the "removed" compound or growth factor, by replacing the previous one.

In other words, the invention relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Culture medium as defined in the method defined above is an aqueous liquid containing minimal essential nutriments required for survival and proliferation of cells, and more generally for maintain the homeostasis of the cell. The culture medium contains, salts, nutriments such as amino acids, nucleotides and ribonucleotides, vitamins... Specifically for carrying out differentiation processes, some specific culture media are available from different manufacturers, these specific media containing some compounds that sustain or help the differentiation process. For instance, mTeSR^{™}1 or mTeSR^{™}Plus media, commercialized by StemCell Biotechnologies, or X-VIVO^{™} 10 medium available from Lonza can be used. The skilled person would be able to choose the most relevant medium for carrying out the above-mentioned steps. Preferably, step a) is carried out with a first culture medium, and the other steps are carried out with a second culture medium which is different from the second culture medium.

All the steps are all carried out in a cell incubator, wherein the temperature is maintained around 37°C, and with 5% CO₂, with a constant humidity rate. These conditions are classical condition for culturing cells and cell lines, and for carrying out a differentiation process.

Advantageously, the invention relates to the above-mentioned method, wherein the Activin A growth factor is used at a concentration of 100 ng/mL in step b) and at a concentration of 50 ng/mL at steps c) and d).

Thus, the invention advantageously relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention relates to the above defined method, wherein the Y27 compound and the FGF10, IGF1 and EGF growth factors are used at a concentration of 10 µM), i.e. 10 µmol/L or 10 10⁻⁶ mol/L.

Thus, the invention relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Thus, the invention advantageously relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to a method as defined-above, wherein the CHIR99 growth factor is used at a concentration of 5µM.

More advantageously, the invention relates to a method as defined-above, wherein the Noggin growth factor is used at a concentration of 100 ng/mL.

Advantageously, the invention relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, 100 ng/mL of 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention relates to the method as defined above, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

The invention more advantageously relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the method as defined above, wherein the BMP4 growth factor is use in steps g)-h) at a concentration of 10ng/mL and in steps i)-j) at a concentration of 50 ng/mL.

Advantageously, the invention relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL** a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention advantageously relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Thus, the invention relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Thus, the invention advantageously relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, 100 ng/mL of 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention relates to the method as defined above, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

The invention more advantageously relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the method as defined above, wherein the retinoic acid is used in steps e) and f) at a concentration of 0.75 µM.

More advantageously, the invention relates to the method as defined above, wherein the retinoic acid is used in steps g)-j) at a concentration of 0.1µM.

Advantageously, the invention relates to the method as defined above, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the method as defined above, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the method as defined above, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the method as defined above, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the method as defined above, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, 100 ng/mL of 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention relates to the method as defined above, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

The invention more advantageously relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL** a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention advantageously relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and 0.75 µM of a retinoic acid compound, to obtain the cell in a RA /F8 culture medium,
f) removing the RA /F8 culture medium from the cell, and incubating for 48h the cell in a sixth medium supplemented with a FGF8 growth factor, 0.75 µM of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Thus, the invention relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Thus, the invention advantageously relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

Advantageously, the invention relates to the above method, the method comprising:
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, 100 ng/mL of 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, a FGF 10 growth factor, a IGF1 growth factor and an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention relates to the method as defined above, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and 10 µM of a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

The invention more advantageously relates to the above method, the method comprising:
a) incubating the iPSc in a first culture medium supplemented with 10 µM of a Y27 compound, to obtain a cell in a Y27 culture medium, for 24h,
b) removing the Y27 culture medium from the cell, and incubating for 24h the cell with a second culture medium supplemented with 100 ng/mL of an Activin A growth factor and 5µM of a CHIR99 compound, to obtain the cell in an ActA/CHIR99 culture medium,
c) removing the ActA/CHIR99 culture medium from the cell, and incubating for 48h the cell with a third culture medium supplemented with 50 ng/mL of an Actinin A growth factor to obtain the cell in an ActA culture medium,
d) removing the ActA culture medium from the cell, and incubating for 24h the cell in a fourth medium supplemented with 50 ng/mL of an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with a FGF8 growth factor, **0.75 µM** of a retinoic acid compound, 100 ng/mL of a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, **10ng/mL of** a BMP4 growth factor, a LY3 compound and 5µM of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, **10ng/mL of** a BMP4 growth factor and 5µM of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, **50ng/mL of** a BMP4 growth factor and 5µM of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, **50ng/mL of** a BMP4 growth factor, and 10 µM of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention relates to the method as defined above, wherein the FGF8 growth factor is used in steps e) and f) at a concentration of 50 ng/mL, in step g) and h at a concentration of 20 ng/mL, and at step j) at a concentration of 10 ng/mL.

Finally, more advantageously, the invention relates to a method as defined above, the method comprising:
a) incubating the iPSc in a first culture medium supplemented with **10 µM** of a Y27 compound, to obtain a cell in a Y27 culture medium, for 24h,
b) removing the Y27 culture medium from the cell, and incubating for 24h the cell with a second culture medium supplemented with **100 ng/mL** of an Activin A growth factor and **5µM** of a CHIR99 compound, to obtain the cell in an ActA/CHIR99 culture medium,
c) removing the ActA/CHIR99 culture medium from the cell, and incubating for 48h the cell with a third culture medium supplemented with **50 ng/mL** of an Actinin A growth factor to obtain the cell in an ActA culture medium,
d) removing the ActA culture medium from the cell, and incubating for 24h the cell in a fourth medium supplemented with **50 ng/mL** of an Actinin A growth factor and a **10 µM** of Y27 compound, to obtain the cell in a Y27/ActA culture medium,
e) removing the Y27/ActA culture medium from the cell, and incubating for 24h the cell in a fifth medium supplemented with **50 ng/mL** of a FGF8 growth factor and **0.75 µM** of a retinoic acid compound, to obtain the cell in a RA /F8 culture medium,
f) removing the RA /F8 culture medium from the cell, and incubating for 48h the cell in a sixth medium supplemented with **50 ng/mL** of a FGF8 growth factor, **0.75 µM** of a retinoic acid compound, **100 ng/mL** of a Noggin growth factor and **10 µM** of a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with **20 ng/mL** of a FGF8 growth factor, **0.1 µM** of a retinoic acid compound, **10ng/mL** of a BMP4 growth factor, **5 µM** of a LY3 compound and **5µM** of a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with **20 ng/mL** of a FGF8 growth factor, **0.1 µM** of a retinoic acid compound, **10ng/mL** of a BMP4 growth factor and **5µM** of a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with **10 ng/mL** of a FGF8 growth factor, **0.1 µM** of a retinoic acid compound, **50ng/mL** of a BMP4 growth factor and **5µM** of a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with **10 ng/mL** of a FGF8 growth factor, **50ng/mL** of a BMP4 growth factor, and **10 µM** of a FGF 10 growth factor, of a IGF1 growth factor and of an EGF growth factor, to obtain the cells in a RA/F8/BMP/F10/IGF1/EGF culture medium, and
k) removing the RA/F8/BMP/F10/IGF1/EGF culture medium from the cell, and recovering the cell which is the functional TEP.

More advantageously, the invention relates to the method as defined above, wherein wherein the functional TEP cell expresses *FOXN1* and *PAX9* genes, and is EPCAM+CD205+.

The TEP cells obtained according to the process as defined above express specific membrane markers EPCAM and CD205, and express the genes FOXN1 and PAX9.

***FOXN1*** gene codes for the Forkhead box protein N1, a DNA-binding transcription factor that regulate keratin gene expression. Depletion, or knock-out of FOXN1 gene leads to an athymic phenotype. FOXN1 has been shown, in thymus, to bind to and regulate genes involved in T-cell maturation and antigen presentation.

***PAX9*** gene codes for Paired box gene 9 protein also called PAX9. It has been shown that thymopoiesis requires Pax9 function in thymic epithelial cells.

Epithelial cell adhesion molecule **(EpCAM)** is a transmembrane glycoprotein mediating Ca2+-independent homotypic cell-cell adhesion in epithelia.

**CD205** is an endocytic receptor that is expressed at high levels by cortical thymic epithelial cells and by dendritic cell (DC) subsets.

The invention also relates to a functional TEP cell, obtainable or directly obtained by the method as defined above, said cells expressing *FOXN1* and *PAX9* genes, and being EPCAM+CD205+.

These cells are novel, since they derive from non-naturally occurring stem cells (i.e. iPSC) even if they harbor the same properties and the same differentiation abilities compared to the natural TEC obtained from the differentiation of cells originating from fertilized egg.

Despite from specific markers as defined above, the cells cannot be exhaustively described except by the process allowing to obtain them.

In one other aspect of the invention, it is disclosed here a method for obtaining a thymic epithelial cell from a functional TEP as defined above, the method comprising
a) Incubating the functional TEP in a culture medium supplemented with L-Glutamine or the like and a first composition comprising BMP4, FGF8, FGF10, IGF1 and EGF growth factors and second composition comprising RANKL, IL7, FTL3 and SCF growth factors for 96h, to obtain a first TEP differentiation culture,
b) removing from the first TEP differentiation culture the first composition for 120h, in order to obtain second TEP differentiation culture, and
c) Recovering the thymic epithelial cell from second TEP differentiation culture.
Advantageously, in Step b, RANKL can be added two times a week.

In other words, the invention relates to a method for obtaining a thymic epithelial cell from a functional TEP as defined above, the method comprising:
a) Incubating for 96h the functional TEP in a culture medium supplemented with L-Glutamine or the like and a first composition comprising a BMP4, a FGF8, a FGF10, a IGF1 and a EGF growth factors and second composition comprising a RANKL, an IL7, a FTL3 and a SCF growth factors, to obtain a cell in a first TEP differentiation culture medium,
b) removing the cell from the first TEP differentiation culture, and contacting for 120h the cell with a culture medium supplemented with the first composition, in order to obtain the cell in a second TEP differentiation culture medium, and
c) removing second TEP differentiation culture from the cell, and recovering the cell which is the functional the thymic epithelial cell.

More advantageously, the invention relates to the method as defined above, the method comprising:
a) Incubating for 96h the functional TEP in a culture medium supplemented with L-Glutamine or the like and a first composition comprising 50 ng/mL of a BMP4, 10 ng/mL of a FGF8, 10 ng/mL of a FGF10, 10 ng/mL of a IGF1 and 10 ng/mL of a EGF growth factors and second composition comprising 50 ng/mL of a RANKL, 5 ng/mL of a IL7, 5 ng/mL of a FTL3 and 10 ng/mL of a SCF growth factors, to obtain a cell in a first TEP differentiation culture medium,
b) removing the cell from the first TEP differentiation culture, and contacting for 120h the cell with a culture medium supplemented with the second composition, the second composition comprising 50 ng/mL of a RANKL, 5 ng/mL of a IL7, 5 ng/mL of a FTL3 and 10 ng/mL of a SCF growth factors, in order to obtain the cell in a second TEP differentiation culture medium, and
c) removing second TEP differentiation culture from the cell, and recovering the cell which is the functional the thymic epithelial cell.

More advantageously, the invention relates to a method for differentiating, *in vitro* or *ex vivo,* an iPSc into a functional TEC, the method comprising the following steps :
a) incubating the iPSc in a first culture medium supplemented with a Y27 compound, to obtain a cell in a Y27 culture medium, for 24h,
b) removing the Y27 culture medium from the cell, and incubating for 24h the cell with a second culture medium supplemented with an Activin A growth factor and a CHIR99 compound, to obtain the cell in an ActA/CHIR99 culture medium,
c) removing the ActA/CHIR99 culture medium from the cell, and incubating for 48h the cell with a third culture medium supplemented with an Actinin A growth factor to obtain the cell in an ActA culture medium,
d) removing the ActA culture medium from the cell, and incubating for 24h the cell in a fourth medium supplemented with an Actinin A growth factor and a Y27 compound, to obtain the cell in a Y27/ActA culture medium,
e) removing the Y27/ActA culture medium from the cell, and incubating for 24h the cell in a fifth medium supplemented with a FGF8 growth factor and a retinoic acid compound, to obtain the cell in a RA /F8 culture medium,
f) removing the RA /F8 culture medium from the cell, and incubating for 48h the cell in a sixth medium supplemented with a FGF8 growth factor, a retinoic acid compound, a Noggin growth factor and a LY3 compound for 48h, to obtain the cell in a RA/F8/NOG/LY3 culture medium,
g) removing the RA/F8/NOG/LY3 culture medium from the cell, and incubating for 48h the cell in a seventh medium supplemented with a FGF8 growth factor, a retinoic acid compound, a BMP4 growth factor, a LY3 compound and a CHIR99 compound, to obtain the cell in a RA/F8/LY3/CHIR99/BMP culture medium,
h) removing the RA/F8/LY3/CHIR99/BMP culture medium from the cell, and incubating for 24h the cell in an eighth medium supplemented with a FGF8 growth factor, a retinoic acid compound, a BMP4 growth factor and a CHIR99 compound, to obtain the cell in a first RA/F8/CHIR99/BMP culture medium,
i) removing the first RA/F8/CHIR99/BMP culture medium from the cell, and incubating for 24h the cell in a nineth medium supplemented with a FGF8 growth factor, a retinoic acid compound, a BMP4 growth factor and a CHIR99 compound to obtain the cell in a second RA/F8/CHIR99/BMP culture medium,
j) removing the second RA/F8/CHIR99/BMP culture medium from the cell, and incubating for 72h the cell in a tenth medium supplemented with a FGF8 growth factor, a BMP4 growth factor, a FGF 10 growth factor, a IGF1 growth factor and an EGF growth factor, to obtain the cells in a RA/F8/BMP/F10/IGF1/EGF culture medium,
k) removing the RA/F8/BMP/F10/IGF1/EGF culture medium from the cell, and recovering the cell which is the functional TEP,
l) Incubating for 96h the functional TEP in a culture medium supplemented with L-Glutamine or the like and a first composition comprising 50 ng/mL of a BMP4, 10 ng/mL of a FGF8, 10 ng/mL of a FGF10, 10 ng/mL of a IGF1 and 10 ng/mL of a EGF growth factors and second composition comprising 50 ng/mL of a RANKL, 5 ng/mL of a IL7, 5 ng/mL of a FTL3 and 10 ng/mL of a SCF growth factors, to obtain a cell in a first TEP differentiation culture medium,
m) removing the cell from the first TEP differentiation culture, and contacting for 120h the cell with a culture medium supplemented with the second composition, the second composition comprising 50 ng/mL of a RANKL, 5 ng/mL of a IL7, 5 ng/mL of a FTL3 and 10 ng/mL of a SCF growth factors, in order to obtain the cell in a second TEP differentiation culture medium, and
n) removing second TEP differentiation culture from the cell, and recovering the cell which is the functional the thymic epithelial cell.

The invention also relates to a thymic epithelial cell obtainable or directly obtained by the method as defined above.

Advantageously, the invention relates to the thymic epithelial cell as defined above, said cells expressing *AIRE, PSMB11* and *HLA-DRA* genes, along with FOXN1 and PAX9 genes and are EPCAM+CD205+.

The invention also relates to a is a method of treating or preventing a disease of the thymus in a subject in need thereof comprising the steps of administering, transplanting or grafting a therapeutically effective amount of the TEPs or TECs as defined above, a solution comprising the TEPs or TECs as defined above, a composition comprising the TEPs or TECs as defined above of the present disclosure, or a pharmaceutical composition comprising the TEPs or TECs as defined above, to the subject in need thereof. The subject is preferably a mammal, and most preferably human.

A further embodiment is a method of treating or preventing an autoimmune disease in a subject in need thereof comprising the steps of administering, transplanting or grafting a therapeutically effective amount of the TEPs or TECs as defined above, a solution comprising the TEPs or TECs as defined above, a composition comprising the TEPs or TECs as defined above, or a pharmaceutical composition comprising the TEPs or TECs as defined above, to the subject in need thereof. The subject is preferably a mammal, and most preferably human.

Another embodiment is a method of recovering or restoring impairment of the function of the thymus in a subject in need thereof comprising the steps of administering, transplanting or grafting a therapeutically effective amount of the TEPs or TECs as defined above, a solution comprising the TEPs or TECs as defined above, a composition comprising the TEPs or TECs as defined above, or a pharmaceutical composition comprising the TEPs or TECs as defined above, to the subject in need thereof. The subject is preferably a mammal, and most preferably human. In some embodiments the impairment is due to injury. In some embodiments, the impairment is due to aging. In some embodiments, the impairment is due to congenital abnormalities.

Yet a further embodiment is a method of reconstituting T cells after a bone marrow transplant in a subject in need thereof comprising the steps of administering, transplanting or grafting a therapeutically effective amount of the TEPs or TECs as defined above, a solution comprising the TEPs or TECs as defined above, a composition comprising the cells of the present disclosure, or a pharmaceutical composition comprising the TEPs or TECs as defined above, to the subject in need thereof. The subject is preferably a mammal, and most preferably human.

Another aspect of the invention relates to a composition comprising
- a TEP cell as defined above, or
- a TEC cell as defined above,
or both
for its use for
- either treating or preventing a disease of the thymus,
- or treating or preventing an autoimmune disease,
- or reconstituting T cells after a bone marrow transplant.

### Brief description of the drawings

The invention will be better understood in view of the following examples and the following figures:

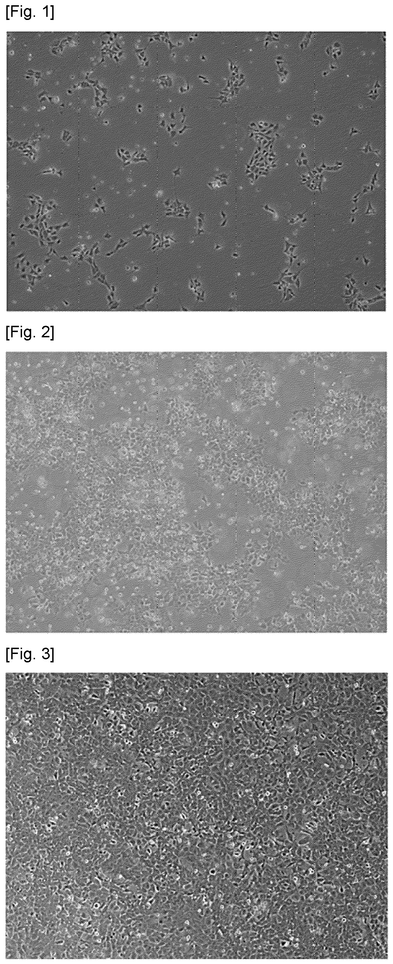

[Fig. 1] Figure 1 represents a photo showing cell morphology at D0 further to induction of differentiation of iPSc.
[Fig. 2] Figure 2 represents a photo showing cell morphology at D1 further to induction of differentiation of iPSc.
[Fig. 3] Figure 3 represents a photo showing cell morphology at D2 further to induction of differentiation of iPSc.

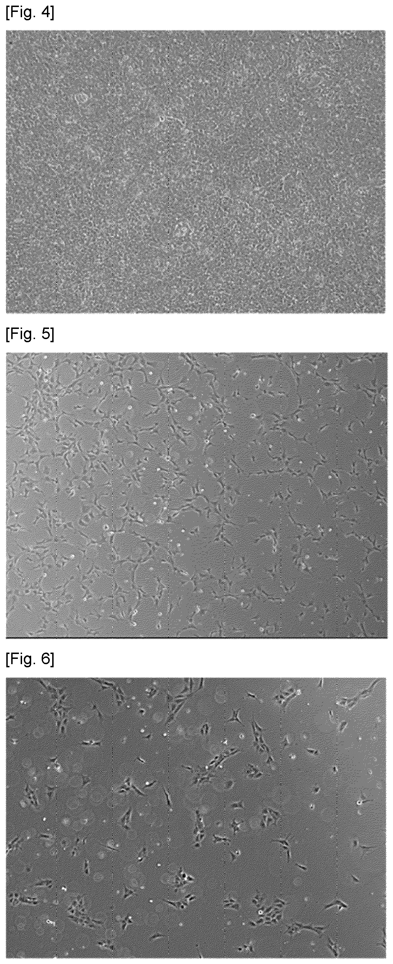

[Fig. 4] Figure 4 represents a photo showing cell morphology at D3 further to induction of differentiation of iPSc.
[Fig. 5] Figure 5 represents a photo showing cell morphology at D4 further to induction of differentiation of iPSc.
[Fig. 6] Figure 6 represents a photo showing cell morphology at D5 further to induction of differentiation of iPSc.

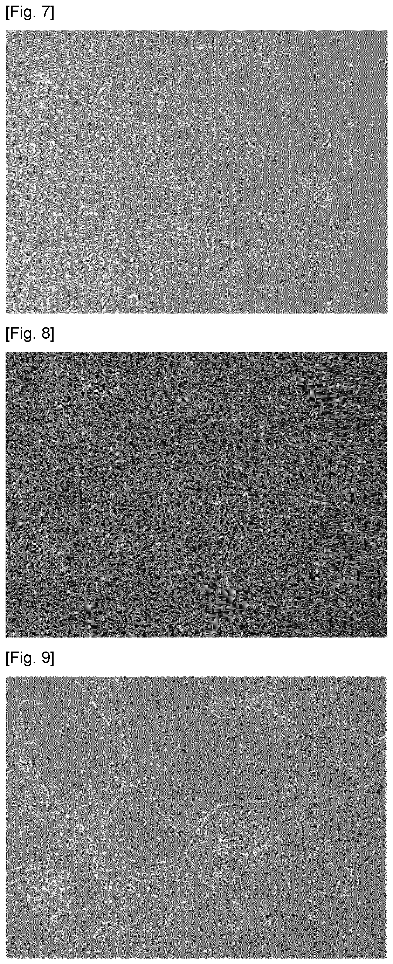

[Fig. 7] Figure 7 represents a photo showing cell morphology at D6 further to induction of differentiation of iPSc.
[Fig. 8] Figure 8 represents a photo showing cell morphology at D7 further to induction of differentiation of iPSc.
[Fig. 9] Figure 9 represents a photo showing cell morphology at D8 further to induction of differentiation of iPSc.

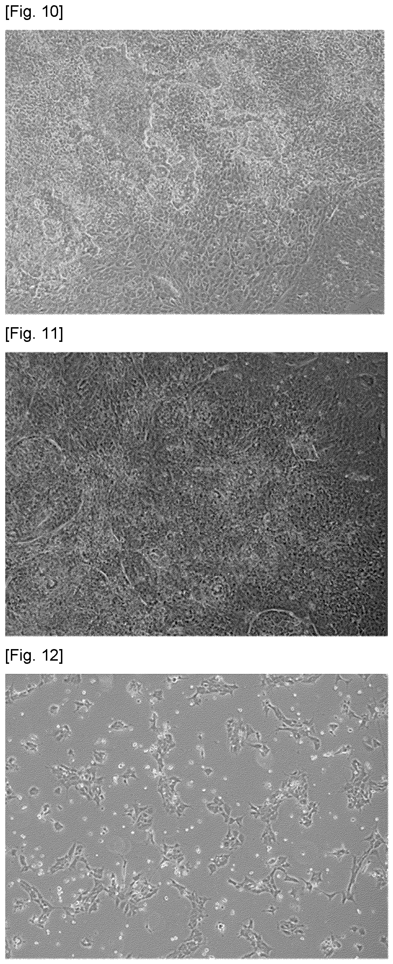

[Fig. 10] Figure 10 represents a photo showing cell morphology at D9 further to induction of differentiation of iPSc.
[Fig. 11] Figure 11 represents a photo showing cell morphology at D10 further to induction of differentiation of iPSc.
[Fig. 12] Figure 12 represents a photo showing cell morphology at D11 further to induction of differentiation of iPSc.
[Fig. 13] Figure 13 represents a photo showing cell morphology at D14 further to induction of differentiation of iPSc.
[Fig. 14] Figure 14 is a graph showing FOXN1 gene expression level relative to GAPDH at Day 0, Day 10 and D14 of iPSc differentiation.
[Fig. 15] Figure 15 is a picture of fluorescent microscopy of iPSCs differentiated during 14 days. The photo shows that FOXN1protein and DNA colocalize in nucleus. Cytoplasm is labelled by keratin 8.
[Fig. 16] Figure 16 is a photo of TEP cells after 1 day with differentiation cocktail.
[Fig. 17] Figure 17 is a photo of TEP cells after 11 days with differentiation cocktail.
[Fig. 18] Figure 18 is a photo of fluorescent microscopy of TEP cells after differentiation toward TEC. The photo shows that DNA (nucleus) and keratin 8 (cytoplasm) do not colocalize.

### Examples

### Example 1 - Differentiation of induced pluripotent stem cells (iPSc) in thymic epithelial progenitors (TEP)

### 1. Purpose

This is a protocol describing the obtention of TEPs from differentiation of iPSc.

The protocol provides technical details on the steps required to generate the TEPs.

It is used in the experiments needing to work on TEPs.

### 2. Process

***Note:*** A « day » of differentiation is defined by a 24h duration. The process being timingdependent, respect of the correct durations is crucial.

### 2.1. Day-1

Prepare *p* culture wells by deposing 500µL Matrigel diluted at 1% in DMEM:F12 (1:1 mixture of DMEM and Ham's F-12 - ThermoFisher Scientifc) and incubate at least 3h at 37°C.

Use an iPSc culture within 60-80% confluency and without spontaneous differentiation.

Wash iPSc with DPBS-/- (Potassium Chloride (KCI) 200 mg/L, Potassium Phosphate monobasic (KH2PO4) 200.0 mg/L, Sodium Chloride (NaCl) g/L and Sodium Phosphate dibasic (Na2HPO4-7H2O) 2.160 g/L, without calcium and magnesium)), remove the DPBS-/- , add 500µL TrypLE per well and incubate 5' at 37°C.

Add 500µL mTeSER1 (StemCell Technologies) per well. Flush multiple times to dissociate all the cells.

Spin 5' at 200g, remove the supernatant and resuspend the cell pellet in 1mL mTESR1.

Count the cells.

Prepare the D-1 cell suspension: pipette a volume of the cell suspension containing 130 000 * *p* cells, complete to *p* mL of mTESR1, add Y27 to a final concentration of 10µM.

Deposit 1mL of D-1 cell suspension per well, homogenize and put in incubator at 37°C and 5% CO₂.

### 2.2. Day 0

Prepare the D0 culture medium: basis XVIVO10, 5µM CHIR99, 100ng/mL ActivinA.

Remove the culture medium in the wells.

Wash with DPBS-/-.

Add 1mL of D0 culture medium per well.

### 2.3. Day 1

Prepare the D1 culture medium: basis XVIVO10, 50ng/mL ActivinA.

Remove the culture medium in the wells.

Wash with DPBS-/-.

Add 1mL of D1 culture medium per well.

### 2.4. Day 2

Prepare the D1 culture medium: basis XVIVO10, 50ng/mL ActivinA.

Remove the culture medium in the wells.

Wash with DPBS-/-.

Add 1mL of D1 culture medium per well.

Prepare q culture wells by deposing 500µL Matrigel diluted at 1% in DMEM:F12 and incubate at least 3h at 37°C.

Cells must be reaching confluency.

### 2.5. Day 3

Wash cells with DPBS-/-, remove the DPBS-/-, add 500µL TrypLE per well and incubate 5' at 37°C.

Add 500µL TrypLE per well. Flush multiple times to dissociate all the cells.

Spin 5' at 200g, remove the supernatant and resuspend the cell pellet in 1mL XVIVO10.

Count the cells.

Prepare the D3 cell suspension (see table): pipette a volume of the cell suspension containing 50 000 * *q* cells, complete to *q* mL of XVIVO10, add ActivinA to a final concentration of 50ng/mL and Y27 to a final concentration of 10µM.

Deposit 1mL of D3 cell suspension per well, homogenise and put in incubator at 37°C and 5% CO₂.

### 2.6. Day 4

Prepare D4 medium: basis XVIVO10, 0.75µM of retinoic acid and 50 ng/mL FGF8.

Remove the culture medium in the wells.

Wash with DPBS-/-.

Add 1mL of D4 culture medium per well.

### 2.7. Day 5

Prepare D5 medium: basis XVIVO10, 0.75µM of retinoic acid, 50 ng/mL FGF8, 10 µM LY3, 100 ng/mL Noggin.

Remove the culture medium in the wells.

Wash with DPBS-/-.

Add 1mL of D5 culture medium per well.

### 2.8. Day 7

Prepare D7 medium: basis XVIVO10, 0.1µM) retinoic acid, 5µM CHIR99, 5 µM LY3, 10 ng/mL BMP4 and 20 ng/mL FGF8.

Remove the culture medium in the wells.

Wash with DPBS-/-.

Add 1mL of D7 culture medium per well.

### 2.9. Day 9

Prepare D9 medium: basis XVIVO10, 0.1µM) retinoic acid, 5µM CHIR99, 10 ng/mL BMP4 and 20 ng/mL FGF8.

Remove the culture medium in the wells.

Wash with DPBS-/-.

Add 1mL of D7 culture medium per well.

Prepare r culture wells by deposing 500µL Matrigel diluted at 1% in DMEM:F12 and incubate at least 3h at 37°C.

### 2.10. Day 10

If the cells haven't reach confluency yet, wait before passing them.

Add 500µL TrypLE per well. Flush multiple times to dissociate all the cells.

Spin 5' at 200g, remove the supernatant and resuspend the cell pellet in 1mL XVIVO10.

Count the cells.

Prepare the D10 cell suspension (see table): pipette a volume of the cell suspension containing 100 000 * *r* cells, complete to r mL of XVIVO10, add 0.1µM retinoic acid, 5µM CHIR99, 50 ng/mL BMP4 and 20 ng/mL FGF8.

Deposit 1mL of D10 cell suspension per well, homogenise and put in incubator at 37°C and 5% CO₂.

### 2.11. Day 11

Prepare D11 medium: basis XVIVO10, 0.1µM retinoic acid, 50 ng/mL BMP4, 10 ng/mL FGF8, 10 ng/mL FGF10, 10 ng/mL IGF1, 10 ng/mL EGF.

Remove the culture medium in the wells.

Wash with DPBS-/-.

Add 1mL of D11 culture medium per well.

### 2.12. Day 13

Prepare D13 medium : basis XVIVO10 0.1µM) retinoic acid, 50 ng/mL BMP4, 10 ng/mL FGF8, 10 ng/mL FGF10, 10 ng/mL IGF1, 10 ng/mL EGF.

Remove the culture medium in the wells.

Wash with DPBS-/-.

Add 1mL of D14 culture medium per well.

### 2.13. Day 14

TEPs are ready to be harvested.

Perform a lysis in a culture well to verify *FOXN1* and *PAX9* expression by RT-qPCR.

Use a culture well to verify differentiation yield: >50% of EPCAM+CD205+ cells.

The above protocol allows the production of TEPs from iPSc for follow-up steps of maturation

The following table summarizes the above mentioned steps :

**[Table 1]**

| **Day** | | **-1** | **0** | **1** | **2** | **3** | **4** | **5** | **6** | **7** | **8** | **9** | **10** | **11** | **12** | **13** |
|---|---|---|---|---|---|---|---|---|---|---|---|---|---|---|---|---|
| **Medium** | | M | X | X | X | X | X | X | X | X | X | X | X | X | X | X |
| **Compound** | UNIT | | | | | | | | | | | | | | | |
| **Y27** | µM | **10** | | | | **10** | | | | | | | | | | |
| **ActA** | ng/mL | | **100** | **50** | **50** | **50** | | | | | | | | | | |
| **NOG** | ng/mL | | | | | | | **100** | **100** | | | | | | | |
| **RA** | µM | | | | | | **0.75** | **0.75** | **0.75** | **0.1** | **0.1** | **0.1** | **0.1** | **0.1** | **0.1** | **0.1** |
| **LY3** | µM | | | | | | | **10** | **10** | **5** | **5** | | | | | |
| **CHIR99** | µM | | | | | | | | | **5** | **5** | **5** | **5** | | | |
| **BMP4** | ng/mL | | **5** | | | | | | | **10** | **10** | **10** | **50** | **50** | **50** | **50** |
| **FGF8** | ng/mL | | | | | | **50** | **50** | **50** | **20** | **20** | **20** | **10** | **10** | **10** | **10** |
| **FGF10** | ng/mL | | | | | | | | | | | | | **10** | **10** | **10** |
| **IGF1** | ng/mL | | | | | | | | | | | | | **10** | **10** | **10** |
| **EGF** | ng/m | | | | | | | | | | | | | **10** | **10** | **10** |

| | | | | | | | | | | | | | | | | |
|---|---|---|---|---|---|---|---|---|---|---|---|---|---|---|---|---|
| M= mTESR1 medium; X= X-VIVO 10 medium. | | | | | | | | | | | | | | | | |

### Example 2 - Maturation of thymic epithelial progenitors (TEP) in functional thymic epithelial cells (TEC) and formation of thymic organoids

### 1. Objective

The following protocol describes the maturation of TECs in a functional thymic organoid obtained from iPSc-derived TEPs, as disclosed in Example 1.

The protocol provides technical details on the steps required to generate the thymic organoids. It is used in the experiments needing to work on thymic organoids.

### 2. Process

Note: Early thymic progenitors (ETP) originate here from primary samples of thymuses from young patients undergoing cardiac surgery the same day.

ETPs are Bone marrow-originating hematopoietic stem cells giving rise to the T cell lineage, with a phenotype: CD45+CD3-CD4-CD8-CD14-CD19-CD56-CD34+CD7+.

### 2.1. Day 0

Check the quality of D14 TEP culture. Cells must be at confluence, forming a dense monolayer with ridges and bulges.

Prepare washing buffer: PBS, 0.5% BSA, 2mM EDTA.

Prepare FACS buffer: PBS, 2% FBS, 5mM EDTA.

Prepare beads for ETP isolation: pipette 1mL anti-mouse Dynabeads, wash 1' with 2mL washing buffer, place on Dynabead Magnet for 2'. Remove supernatant, resuspend beads in 500µL washing buffer. Label beads with Mouse anti-human CD3 antibody for 45' at 40°C under agitation. Wash 3 times with 20mL washing buffer on the Dynabeads magnet.

Resuspend in 20mL washing buffer.

Isolate ETP from a donor primary thymus sample: dissect the thymus in a 50mL Falcon in RPMI medium into 1 cubic millimeter chunks. Let sediment, collect the supernatant, filter through a 70µm mesh. Repeat 3 times. Centrifugate 5' at 200g. Resuspend pellet in 10mL Red Blood Cells Lysis Buffer. Incubate 5' at room temperature. Add 20 mL PBS and centrifuge 5' at 200g. Resuspend pellet in 10mL washing buffer. Count the cells. Collect 1 billion cells and adjust volume to 20mL with washing buffer.

Add to the Dynabeads and incubate 30' at 40°C under agitation.

Place the tube on the magnet for 2'. Collect the supernatant and centrifuge 5' at 200g. Resuspend pellet in 1mL FACS buffer. Label cells with Lin (CD3 CD4 CD8 CD14 CD19 CD56), CD7 and CD34.

Sort cells with a flow cytometer. Centrifuge 5' at 200g. Resuspend pellet in 1mL XVIV010 (LONZA ref# 04-380Q) buffer. Count the cells.

Wash TEPs with DPBS-/-, remove the DPBS-/-, add 500µL TrypLE (ThermoFischer Scientific; recombinant Trypsin, devoid of any cell and animal contaminents) per well and incubate 5' at 37°C.

Add 500µL XVIVO10 per well. Flush multiple times to dissociate all the cells.

Spin 5' at 200g, remove the supernatant and resuspend the cell pellet in 1mL XVIVO10.

Count the cells.

Pool the 2 cellular suspensions and adjust volume with XVIVO10 to a concentration of 200 000 TEP / mL and 50 000 ETP / mL.

Add D14 supplements at the desired concentration (see Table 2), i.e.: 50ng/mL BMP4, 10 ng/mL FGF8, 10 ng/mL FGF10, 10 ng/mL IGF1, 10 ng/mL EGF, 50 ng/mL RANKL, 5 ng/mL IL-7, 5 ng/mL FLT3, 10 ng/mL SCF and 1% Glutamax.

Homogenize and plate in a Low Binding 96-wells plate, at 100µL per well. Put in incubator at 37°C and 5% CO₂.

### 2.2. Day 1

Prepare the hydrogels: unfreeze on ice aliquots of Thrombin (10U/mL) and Aprotinin (26000U/mL) solutions. Do not vortex. Unfreeze fibrinogen (8mg/mL) in a hot water bath at 37°C. Do not vortex.

Flush slowly the aliquots to homogenize. Prepare 0.25 x o eppendorfs tubes, where o is the number of organoids seeded the day before. Add in each tube 150µL Fibrinogen and 10µL Aprotinin. Add 150µL Thrombin in a tube, flush 2 times quickly and without generating bubbles, then pipette 150µL and put in an insert of a 24-wells Hanging Insert plate. Quickly repeat with the remaining 150µL in a second well. Avoid forming bubbles by careful handling of the pipette.

When all wells of a plate are casted, incubate 1 hour at 37°C. The transparent liquid solution with solidify and become opaque.

Prepare the D15 solution, i.e. 50ng/mL BMP4, 10 ng/mL FGF8, 10 ng/mL FGF10, 10 ng/mL IGF1, 10 ng/mL EGF, 50 ng/mL RANKL, 5 ng/mL IL-7, 5 ng/mL FLT3, 10 ng/mL SCF and 1% Glutamax.

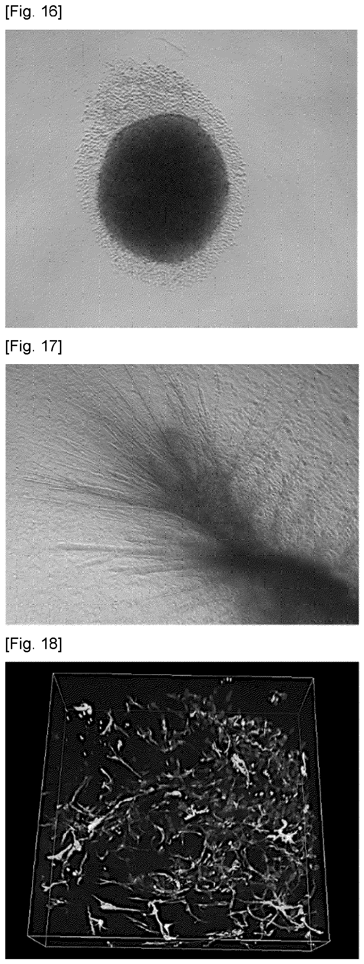

Seed the organoids: verify the quality of the reaggregation step, the organoids must form spheric cell masses with a compact core surrounded by a less dense thymocyte crown (see **Figure** 16).

Using a tip-cut P200 cone previously washed with anti-adherent solution, delicately collect the organoids and seed them at the top of the hydrogels one by one, with an amount of 2 organoids per well. Check that no cells are left in the P96 wells.

Slowly add 300µL of D15 solution at the top of the hydrogels, without directly touching them. Add 700µL of D15 solution in the bottom of each well. Put in incubator at 37°C and 5% CO₂.

### 2.3. Day 2

Check if the organoids are well seeded: the hydrogels must have stayed in place, and the organoids must not sediment at the bottom of the insert. Leave the D15 solution until.

### 2.4. Day 5

From this point the culture medium can be changed daily or two times a week (The organoids can be maintained up to 6 weeks in culture).

Add D19 solution, i.e. 50 ng/mL RANKL, 5 ng/mL IL-7, 5 ng/mL FTL3, 10 ng/mL SCF and 1% Glutamax.

### 2.5. Day 14

TEPs are ready to be harvested.

Perform a lysis (in RTL Buffer, Qiagen ref# 79216) in a culture well to verify FOXN1 and PAX9 expression by RT-qPCR.

Use a culture well to verify differentiation yield: >50% of EPCAM+CD205+ cells, by flow cytometry by using appropriated antibodies

The following table summarizes the above mentioned steps :

**[Table 2]**

| **Day** | | **14** | **15** | **16** | **17** | **18** | **19** | **20** | **21** | **22** | **23** |
|---|---|---|---|---|---|---|---|---|---|---|---|
| **Medium** | | X | X | X | X | X | X | X | X | X | X |
| **Compound** | UNIT | | | | | | | | | | |
| **Y27** | µM | | | | | | | | | | |
| **ActA** | ng/mL | | | | | | | | | | |
| **NOG** | ng/mL | | | | | | | | | | |
| **RA** | µM | | | | | | | | | | |
| **LY3** | µM | | | | | | | | | | |
| **CHIR99** | µM | | | | | | | | | | |
| **BMP4** | ng/mL | **50** | **50** | **50** | **50** | **50** | | | | | |
| **FGF8** | ng/mL | **10** | **10** | **10** | **10** | **10** | | | | | |
| **FGF10** | ng/mL | **10** | **10** | **10** | **10** | **10** | | | | | |
| **IGF1** | ng/mL | **10** | **10** | **10** | **10** | **10** | | | | | |
| **EGF** | ng/mL | **10** | **10** | **10** | **10** | **10** | | | | | |
| **RANKL** | ng/mL | **50** | **50** | **50** | **50** | **50** | **50** | **50** | **50** | **50** | **50** |
| **IL7** | ng/mL | **5** | **5** | **5** | **5** | **5** | **5** | **5** | **5** | **5** | **5** |
| **FTL3** | ng/mL | **5** | **5** | **5** | **5** | **5** | **5** | **5** | **5** | **5** | **5** |
| **SCF** | ng/mL | **10** | **10** | **10** | **10** | **10** | **10** | **10** | **10** | **10** | **10** |
| **Glutamax** | | **1%** | **1%** | **1%** | **1%** | **1%** | **1%** | **1%** | **1%** | **1%** | **1%** |

| | | | | | | | | | | | |
|---|---|---|---|---|---|---|---|---|---|---|---|
| X= X-VIVO 10 medium. | | | | | | | | | | | |

### 3. Results

Fig 17 shows the structural changes of thymic organoids in culture in the hydrogel. Organoids shift from a compact spheric form [Fig 16] to a larger and less dense structure formed of elongated cells. This structural change echoes the *in vivo* 3D structure of the thymic epithelium, a sponge-like mesh of low density. By using IF and 3D imaging we highlight this structure in the organoids, using cytokeratin8 (KRT8) to label TECs.

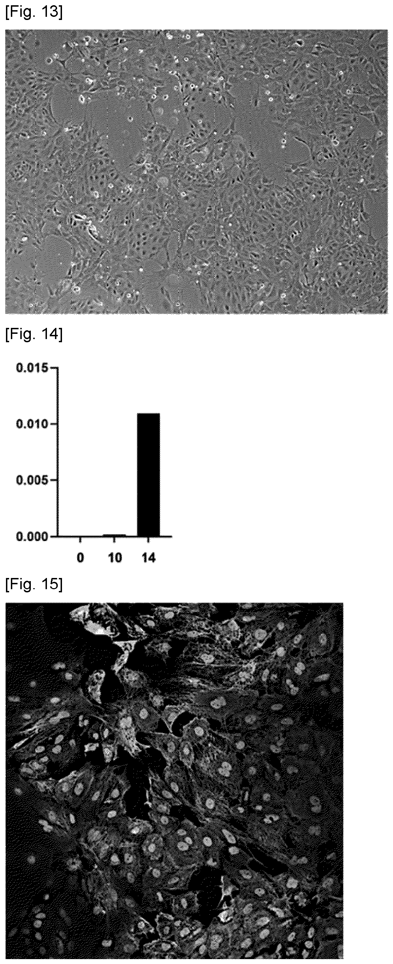

The obtained TEP express the expected markers of thymic epithelium identity such as FOXN1 and PAX9. Moreover, TEP morphology is similar to primary cells with large polygonal cells [Fig 15]. Thus, this process allows the generation of primary-like TEP from iPSc.

## Claims

1. A use of a composition comprising the following compounds: Activin A, BMP4, CHIR99,
EGF, FGF 8, FGF 10, IGF1, LY3, Noggin, retinoic acid and Y27,
for implementing a differentiation process, preferably *in vitro* or *ex vivo*, of an induced pluripotent stem cell or iPSc, into a functional thymic epithelial progenitor or TEP.

2. The use according to claim 1, wherein the differentiating process takes place for 14 days.

3. The use according to claim 1 or 2, wherein the iPSc expresses *OCT4* and *NANOG* genes

4. A method for differentiating, *in vitro* or *ex vivo*, an iPSc into a functional TEP, the method comprising the following steps:
a) Incubating the iPSc in a culture medium supplemented with a Y27 compound for 24h, to obtain a Y27 culture medium,
b) removing the Y27 compound from the Y27 compound, and adding an Activin A growth factor and a CHIR99 compound for 24h, to obtain an ActA/CHIR99 culture medium,
c) removing the CHIR99 growth from the ActA/CHIR99 culture medium, and maintaining the culture with the Activin A for 48h, to obtain an ActA culture medium,
d) Adding to the ActA culture a Y27 compound for 24h, to obtain a Y27/ActA culture medium,
e) Removing the ActA and the Y27 compound from the Y27/Act1 culture medium, and adding to the Y27/Act1 culture medium a FGF8 growth factor and retinoic acid for 24h, to obtain a RA /F8 culture medium,
f) Adding to the RA/F8 culture medium a Noggin growth factor and a LY3 compound for 48h, to obtain a RA/F8/NOG/LY3 culture medium,
g) Removing the Noggin growth factor from the RA/F8/NOG/LY3 culture medium, and adding a CHIR99 compound and a BMP4 growth factor for 48h, to obtain a RA/F8/LY3/CHIR99/BMP culture medium,
h) Removing from the RA/F8/LY3/CHIR99/BMP culture the LY3 compound for 24h to obtain a RA/F8/CHIR99/BMP culture medium,
i) Modifying the concentration of the BMP4 growth factor and the FGF8 growth factor for 24h in the RA/F8/CHIR99/BMP culture medium,
j) Removing from the RA/F8/CHIR99/BMP culture medium the CHIR99 compound and adding for 72h a FGF10 growth factor, an IGF1 growth factor and an EGF growth factor, to obtain a RA/F8/BMP/F10/IGF1/EGF culture medium, and
k) Recovering the functional TEP from the RA/F8/BMP/F10/IGF1/EGF culture medium, after 72h.

5. The method according to claim 4, wherein the Activin A growth factor is used at a concentration of 100 ng/mL in step b) and at a concentration of 50 ng/mL at steps c) and d).

6. The method according to claim 4, wherein the Y27 compound and the FGF10, IGF1 and EGF growth factors are used at a concentration of 10 µM.

7. The method according to claim 4, wherein the CHIR99 growth factor is used at a concentration of 5µM, and the Noggin growth factor is used at a concentration of 100 ng/mL.

8. The method according to claim 4, wherein the BMP4 growth factor is use in step g)-h) at a concentration of 10ng/mL and in steps i)-j) at a concentration of 50 ng/mL.

9. The method according to step 4, wherein the retinoic acid is used in steps e) and f) at a concentration of 0.75 µM.

10. The method according to step 4, wherein the FGF8 growth factor is used in steps e) and f) at a concentration of 50 ng/mL, in step g) and h at a concentration of 20 ng/mL, and at step j) at a concentration of 10 ng/mL.

11. The method according to anyone of claims 4 to 10, wherein the functional TEP cell expresses *FOXN1* and *PAX9* genes, and is EPCAM+CD205+.

12. A functional TEP cell, obtainable or directly obtained by the method according to anyone of claims 4 to 11, said cells expressing *FOXN1* and *PAX9* genes, and being EPCAM+CD205+ .

13. A method for obtaining a thymic epithelial cell from a functional TEP as defined in claim 12, the method comprising :
a) Incubating the functional TEP in a culture medium supplemented with L-Glutamine and a first composition comprising BMP4, FGF8, FGF10, IGF1 and EGF growth factors and second composition comprising RANKL, IL7, FTL3 and SCF growth factors for 96h, to obtain a first TEP differentiation culture,
b) removing from the first TEP differentiation culture the first composition for 120h, in order to obtain second TEP differentiation culture, and
c) Recovering the thymic epithelial cell from second TEP differentiation culture.

14. A thymic epithelial cell obtainable or directly obtained by the method according to claim 13, preferably, said cells expressing *AIRE, PSMB11* and *HLA-DRA* genes, along with *FOXN1* and *PAX9* genes and are EPCAM+CD205+.
